# EUROPEAN PATENT APPLICATION

(11) **EP 2 793 029 A1**
(43) Date of publication of application: **22.10.2014**
(21) Application number: 12857435.7
(22) Date of filing: 13.12.2012
(51) Int. Cl.: G01N 33/68, G01N 33/53, G01N 33/52, C07K 1/14

(54) **METHOD FOR FINDING BIOACTIVE PEPTIDES**

(30) Priority: 13.12.2011 KR 20110133723
(71) Applicant: Nano Intelligent Biomedical Engineering Corporation Co., Ltd., Seoul 110-749 (KR)
(72) Inventor: CHUNG, Chong-Pyoung, Seoul 138-170 (KR); PARK, Yoon Jeong, Seoul 152-770 (KR); LEE, Jue-Yeon, Gwacheon-si Gyeonggi-do 427-736 (KR)
(74) Representative: Hiebl, Inge Elisabeth
(86) International application number: PCT/KR2012/010839
(87) International publication number: WO 2013/089450

(57) **Abstract**

The present invention relates to a method for discovering bioactive peptides, and a use thereof. The present invention relates to a method for discovering a peptide which has an effect on tissue regeneration or a peptide which adheres to a biomaterial, by searching the smallest bioactive domain from a protein. This method for discovering a peptide is named PEPscovery (**PEP**tide Di**scovery**). According to the present invention, it is possible to discover peptides comprising 20 or more amino acids more rapidly than conventional techniques for discovering a peptide, and to discover bioactive peptides effective in tissue regeneration and capable of being adhered to a specific biomaterial through PEPscovery, thereby developing medical supplies and medical equipment and applying the same to the development of a diagnostic chip.

<110>    Nano Intelligent Biomedical Engineering Corporation. co. Ltd

<120>    The Discovery Method for the Bioacitve Peptide Thereof-Peptide
         Discovery

<130>    PP-B1179

<160>    10

<170>    KopatentIn 1.71

<210>    1
<211>    5
<212>    PRT
<213>    Artificial Sequence

<220>
<223>    AMINO ACID


<400>    1
Ser Pro Lys His His
  1               5


<210>    2
<211>    14
<212>    PRT
<213>    Artificial Sequence

<220>
<223>    AMINO ACID


<400>    2
Ser Gln Arg Ala Arg Lys Lys Asn Lys Asn Cys Arg Arg His
  1               5                  10


<210>    3
<211>    11
<212>    PRT
<213>    Artificial Sequence

<220>
<223>    AMINO ACID


<400>    3
Ser Leu Tyr Val Asp Phe Ser Asp Val Gly Trp
  1               5                  10


<210>    4
<211>    13
<212>    PRT
<213>    Artificial Sequence

<220>
<223>    AMINO ACID


<400>    4

Asn Asp Trp Ile Val Ala Pro Pro Gly Tyr Gln Ala Phe
1               5                   10

<210>   5
<211>   10
<212>   PRT
<213>   Artificial Sequence

<220>
<223>   AMINO ACID

<400>   5
Tyr Cys His Gly Asp Cys Pro Phe Pro Leu
1               5                   10

<210>   6
<211>   15
<212>   PRT
<213>   Artificial Sequence

<220>
<223>   AMINO ACID

<400>   6
Ala Asp His Leu Asn Ser Thr Asn His Ala Ile Val Gln Thr Leu
1               5                   10                  15

<210>   7
<211>   18
<212>   PRT
<213>   Artificial Sequence

<220>
<223>   AMINO ACID

<400>   7
Val Asn Ser Val Asn Ser Ser Ile Pro Lys Ala Cys Cys Val Pro Thr
1               5                   10                  15

Glu Leu

<210>   8
<211>   11
<212>   PRT
<213>   Artificial Sequence

<220>
<223>   AMINO ACID

<400>   8
Ser Ala Ile Ser Met Leu Tyr Leu Asp Glu Tyr
1               5                   10

<210>    9
<211>    11
<212>    PRT
<213>    Artificial Sequence

<220>
<223>    AMINO ACID

<400>    9
Asp Lys Val Val Leu Lys Asn Tyr Gln Glu Met
1               5                   10

<210>    10
<211>    8
<212>    PRT
<213>    Artificial Sequence

<220>
<223>    AMINO ACID

<400>    10
Val Val Glu Gly Cys Gly Cys Arg
1               5

## Description

### TECHNICAL FIELD

The present invention relates to a method for discovering a bioactive peptide, and more particularly to a method for discovering a bioactive peptide, or a peptide which adheres to a biomaterial, by identifying a minimal domain having bioactivity from a protein.

### BACKGROUND ART

Peptide is the smallest function unit of protein that is involved in signaling and functional regulation *in vivo.* Peptide refers to a substance consisting of two or more amino acids linked like a chain, and a short protein or an amino acid polymer consisting of a chain containing 50 or less amino acids is generally defined as peptide. These peptides are important materials that used as bioscience materials in the bio-industry and as therapeutic agents or functional substances in the biomedicine field and the biochemical field.

Peptides can be made *in vivo* by biosynthetic processes from genes and can also be made *in vitro* by amino acid synthesis based on chemical methods. Such peptides can be used in a large range of applications, including diagnosis of diseases by protein-protein interactions, identification of cell differentiation, preparation of medical drugs, agents for diagnosis of diseases, materials for nanomaterials, etc. 23 kinds of amino acids constitute peptides, but the kind of peptides that can be made greatly varies depending on the number of the constituent amino acids, and thus the range of search for bioactive peptides is also very broad. In this possibility, peptides have received attention for the following reasons. Unlike high-molecular-weight proteins or highly fat-soluble substances that are accumulated in the liver or the like when administered *in vivo,* peptides are discharged from the body after action in the body, and thus have no risk of causing toxicity in the body. Further, peptides have less risk of causing side effects of antibodies produced by immune reactions when injected *in vivo,* unlike protein drugs. Also, peptides show high activity due to their specific binding to target substances, may have various molecular structures depending on the kind and number of amino acids, and can be made not only in cells, but also by synthetic methods, and thus can be validated as homogeneous compounds. Due to these advantages, peptides are highly applicable as medical drugs or biomedical materials. In addition, as the costs for development of new drugs based on low-molecular-weight substances or natural substances are increasing and target substances for development of new drugs are being diversified with the development of molecular biology or medical science, peptides are receiving as new substances that cope with these changes. In addition, processes for synthesis of peptides have been continuously developed, and thus these peptides can be easily produced in large amounts compared to other biomedical drugs, and the market of these peptides will grow as the market of new bio-drugs and materials grows.

Methods for discovering such bioactive peptides typically include a phage display technique and a molecular modeling technique. Phage display is a technique of discovering unknown amino acid sequences, which have the ability to bind to specific proteins, using recombinant bacteriophages produced by artificially introducing genes, which produce various amino acid sequences, into the genes of bacteriophages parasitic on bacteria. This technique is used in various applications, including epitope mapping, vaccine development, ligand-receptor affinity research, and bioactive peptide selecting (Smith GP, Scott JK., Libraries of peptides and proteins displayed on filamentous phage, Methods Enzymol, 279:377-380. 1993). It is designed to select phages, which have a strong ability to a specific protein, by a series of processes, including biopanning, using bacteriophages which express different peptides and are obtained by artificially inserting gene sequences into the ends of coat protein-producing genes of the bacteriophage genome so as to express peptides having 5-10 random amino acids and transfecting the bacteriophages into *E. coli.* When genomic DNA is artificially extracted from the selected bacteriophages and the nucleotide sequence of the artificially inserted DNA expressing specific peptides is analyzed, the desired functional peptide can be obtained. However, the process of extracting DNA after proliferation in *E. coli* and analyzing the DNA sequence is highly time-consuming. In addition, the number of amino acids constituting the discovered peptide is about 5-10, and it is difficult to screen peptides having an amino acid length longer than 5-10 amino acids.

Molecular modeling refers to simulation which is performed in an imaginary reality and space using a computer when it is difficult to perform analysis (e.g., determination of transition state) by biochemical experiments or when it is time-consuming to perform all experiments (e.g., drug screening). New drug screening methods can be used to discover bioactive peptides. When a new drug candidate showing activity for any protein is to be screened, excessively large amounts of time and costs are required for the synthesis of all molecules. When a small molecule is docked to the active site of a protein having a known x-ray structure, it is helpful in identifying a potential candidate, even though it is difficult to obtain very detail information. This enables to identify the minimal active site of protein and the amino acid sequence of the active site. However, such computer modeling has a limitation in that the same water molecule as that in the body environment or the flexibility of protein cannot be perfectly used. Thus, computer modeling should be based on experimental data, and the results thereof can be used as references to verify the experimental data. Thus, molecular modeling also has a limitation in identifying peptide sequences in a rapid and accurate manner.

Korean Patent No. 10-0864011 relates to a method for constructing a polypeptide library, and more particularly to a method of constructing a library of polypeptides having different molecular weights, shapes or functional groups by degrading proteins by hydrolase or reagents. However, this method is merely a method of producing peptides by enzymatic degradation and has a limitation in selecting a peptide having a specific function from a library consisting of numerous peptides. In addition, Korean Patent Publication No. 10-2008-0083807 relates to a method of discovering a peptide binding to a specific antigenic protein. In this method, the antigenic protein of *Bacillus anthracis* is bound to a micro well plate, and then a peptide binding to the antigenic protein is discovered using the phage display technique. For this reason, this method has shortcomings in that it is time-consuming due to the use of the phage display technique and in that the number of amino acids constituting the discovered peptide is about 5-10, and it is difficult to discover peptides having an amino acid length longer than 5-10 amino acids.

In addition, in "One Bead, One Peptide" method (Korean society of medical biochemistry & molecular biology news, December, pp. 68) that is a library construction method based on split synthesis, a peptide sequence specific to each resin is constructed by split synthesis using resin, which is based on a polystyrene matrix and uses polyethylene glycol (PEG) as a linker. A sufficient amount (100 pmole) of peptide to sequence is attached to each bead, and a pentapeptide having thereto 19 amino acids excluding cysteine has more than 3,000,000 peptides. It is used for investigation of the epitope of antibody, ligands, etc. However, when the synthesized peptide is a new sequence present or not present in natural protein and the number of libraries is several thousands to several tens of thousands, it is difficult to perform the analysis of physiological activity. To overcome this difficulty, the peptide bound to the bead is analyzed by MALDI-TOF, but the analysis of molecular weight cannot demonstrate that the peptide is biologically active.

In a conventional technique (Samuel J. et al., Mol Cancer Ther, Vol. 3:1439, 2004) of detecting fluorescence caused by the binding between a peptide and a reactant, a resin-fluorescent dye-peptide-quencher system is used to discover a peptide that is hydrolyzed by an enzyme that is frequently present in a specific disease. When the middle portion of the peptide is hydrolyzed by the enzyme, the quencher is cleaved, but fluorescence that is still attached to the resin can be emitted, making accurate analysis difficult.

Accordingly, the present inventors have made extensive efforts to develop a method of discovering bioactive peptides in a rapid and accurate manner, compared to conventional methods of discovering bioactive peptides. As a result, the present inventors have found that the use of a method comprising the following steps can effectively discover a bioactive peptide, thereby completing the present invention: (a) adding bead resin to each well of a well plate, and synthesizing a peptide, which contains a protein fragment having desired bioactivity, on the surface of the bead in each well, thereby constructing a bioactive peptide library; and (b) screening a peptide having desired bioactivity from the bioactive peptide library.

### DISCLOSURE OF INVENTION

### TECHNICAL PROBLEM

It is an object of the present invention to provide a method of discovering a bioactive peptide, or a peptide which adheres to a biomaterial, by identifying a minimal domain having bioactivity from a protein.

### TECHNICAL SOLUTION

To achieve the above object, the present invention provides a method for discovering a peptide, the method comprising the steps of: (a) adding bead resin to each well of a well plate, and synthesizing a peptide, which contains a protein fragment having desired bioactivity, on the surface of the bead in each well, thereby constructing a bioactive peptide library; and (b) screening a peptide having desired bioactivity from the bioactive peptide library.

### BRIEF DESCRIPTION OF THE DRAWINGS

FIG. 1 is a conceptual view of PEPscovery according to the present invention.
FIG. 2 is a schematic view showing a specific method of PEPscovery according to the present invention.
FIG. 3 is a schematic view showing a process of determining the presence of binding between a target substance and a peptide by fluorescence.
FIG. 4 shows the amino acid sequences of BMP-4 peptides obtained by cleavage with chymotrypsin.
FIG. 5 shows the results obtained by measuring the binding of heparin-binding peptides to heparin by s solid phase method.
FIG. 6 shows the results obtained by measuring the cell differentiation potential of heparin-binding peptides.

### BEST MODE FOR CARRYING OUT THE INVENTION

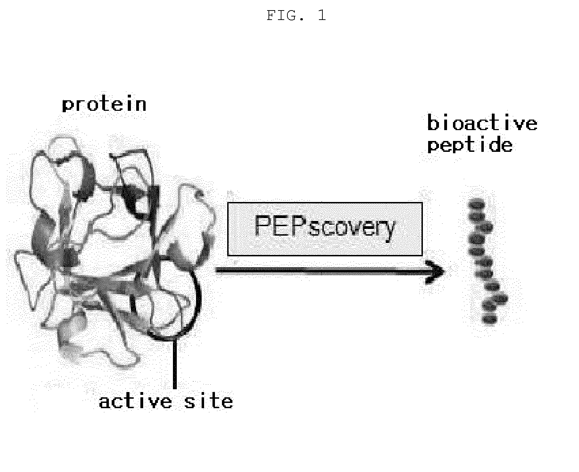

The present invention relates to a method for discovering a bioactive peptide, and more particularly to a method of discovering a bioactive peptide, or a peptide which adheres to a biomaterial, by identifying a minimal domain having bioactivity from a protein. The peptide discovering method is named PEPscovery (**PEP**tide Di**scovery**) (see FIG. 1).

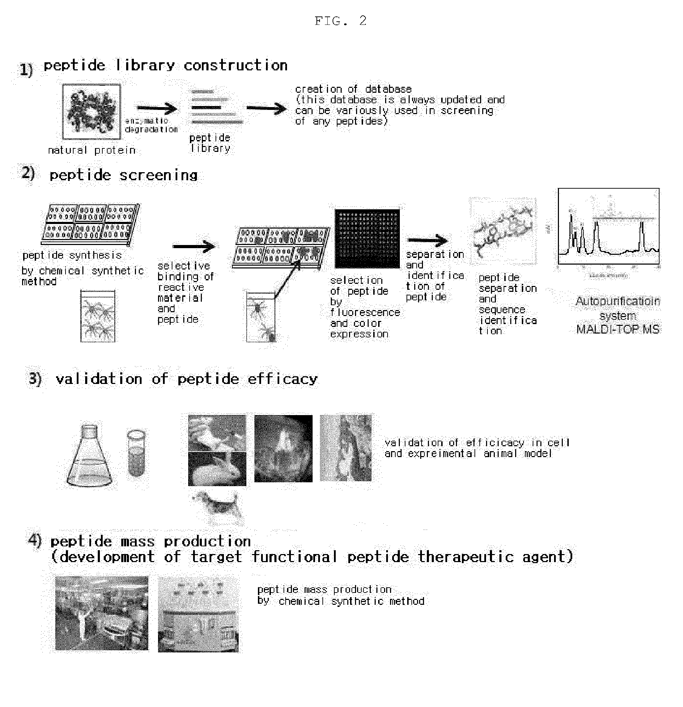

The present invention is directed to a method for discovering a peptide, the method comprising the steps of: (a) adding a resin bead to each well of a well plate, and synthesizing a peptide, which contains a protein fragment having desired bioactivity, on the surface of the bead in each well, thereby constructing a bioactive peptide library; and (b) screening a peptide having desired bioactivity from the bioactive peptide library (see FIG. 2).

In step (a) of constructing the peptide library, natural proteins having known structures, such as active proteins, growth factors, disease-related proteins or intracellular transcription factors, are used. In the method of constructing the peptide library, information is constructed by examining the amino acid sequences of different polypeptides resulting from the degradation of proteins (as described above) by protease, based on search against the known protein data bank. Herein, some amino acids of the amino acid sequences may be substituted with other amino acids having similar physical properties.

In order to select the peptides in step (b), first, the peptide library constructed in step (a) is chemically synthesized and then peptides are synthesized on the surface of the resin as a bead type, according to each amino acid sequence. This bead synthesizes each kind of peptides on a chip in divided well and thus can synthesize various peptides as many as the number of wells. After completion of synthesis of the peptides, a target substance is reacted with the peptides. Examples of the target substance include proteins (tissue growth factors, bone morphogenetic proteins, extracellular matrix proteins, etc.) related to the regeneration of bone, nerve or blood vessels, receptors for the proteins, disease-causing proteins (TNF-α, interleukin, etc.), biomaterials for tissue regeneration (collagen, chitosan, heparin, calcium phosphate, etc.), and the like.

When the target substance to be reacted is a protein, an antibody for the protein is reacted. The antibody is preferably phosphatase- or peroxidase-conjugated antibody. When alkaline phosphatase is used, a solution of BCIP (5-bromo-4-chloro-3-indolyl phosphate p-toluidine salt) and NBT (nitro-blue tetrazolium chloride) in PBS is reacted with the bead. The bead having the phosphorylated antibody bound thereto changes its color to a clear red, and then the bead is washed to stop the reaction. When peroxidase is used, the bead may be reacted with ABTS (2,2'-azinobis [3-ethylbenzothiazoline-6-sulfonic acid]-diammonium salt) to observe a change in the color.

When an antibody for the target substance to be reacted does not exist, a quenching dye and a fluorescent substance (FITC or rhodamine) may be bound to the target substance. The fluorescent substance and the quenching dye are bound using a cross-linking agent.

Examples of the cross-linking agent that can be used in the present invention include, but not limited to, 1,4-bis-maleimidobutane (BMB), 1,11-bis-maleimidotetraethyleneglycol (BM[PEO]4), 1-ethyl-3-[3-dimethyl aminopropyl] carbodiimide hydrochloride (EDC), succinimidyl-4-[N-maleimidomethylcyclohexane-1-carboxy-[6-amidocaproate]] (SMCC) and its sulfonate (sulfo-SMCC), succimidyl 6-[3-(2-pyridyldithio)-ropionamido] hexanoate (SPDP) and its sulfonate (sulfo-SPDP), m-maleimidobenzoyl-N-hydroxysuccinimide ester (MBS) and its sulfonate (sulfo-MBS), and succimidyl[4-(p-maleimidophenyl) butyrate] (SMPB) and its sulfonate (sulfo-SMPB).

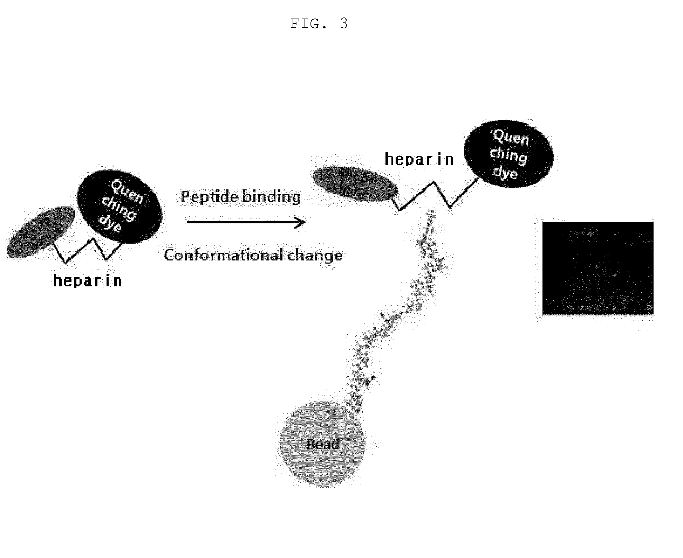

In this state, the fluorescence of the fluorescent substance does not appear due to the quenching dye, and if the synthesized peptide on the bead has binding affinity for the target substance, the fluorescence will be expressed due to a conformational change (see FIG. 3). Thus, the presence of binding can be determined by the expression of fluorescence. A bead that showed either a change in color or fluorescence is selected, and the peptide is isolated from the selected bead. The molecular weight and amino acid sequence of the isolated peptide are analyzed.

The effect of the peptide selected in step (b) is tested using established cell and animal models in order to verify whether the peptide has a tissue regenerating effect. The effect of the peptide is verified using various tissue models, including bone tissue, neuronal tissue, tooth tissue, and blood vessel tissue.

### EXAMPLES

Hereinafter, the present invention will be described in further detail with reference to examples. It will be obvious to a person having ordinary skill in the art that these examples are illustrative purposes only and are not to be construed to limit the scope of the present invention.

### Example 1: Screening of heparin-binding peptide from bone morphogenetic proteins

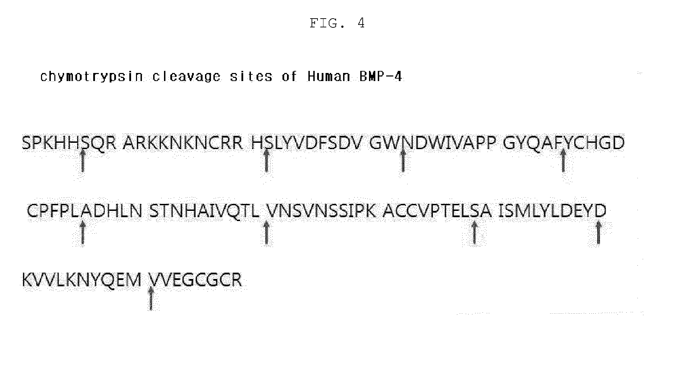

With reference to the chymotrypsin cleavage sites of BMP-4 in the protein data bank, 10 kinds of peptides were selected and synthesized (FIG. 4).
[SEQ ID NO: 1]: SPKHH
[SEQ ID NO: 2]: SQRARKKNKNCRRH
[SEQ ID NO: 3]: SLYVDFSDVGW
[SEQ ID NO: 4]: NDWIVAPPGYQAF
[SEQ ID NO: 5]: YCHGDCPFPL
[SEQ ID NO: 6]: ADHLNSTNHAIVQTL
[SEQ ID NO: 7]: VNSVNSSIPKACCVPTEL
[SEQ ID NO: 8]: SAISMLYLDEY
[SEQ ID NO: 9]: DKVVLKNYQEM
[SEQ ID NO: 10]: VVEGCGCR

Resin (0.075 mmol/g, 100-200 mesh, 1% DVB crosslinking) having bound thereto Fmoc-(9-fluorenylmethoxycarbonyl) as a blocking group was added to each well of a plate and swollen with DMF. Then, the resin was treated with 20% piperidine/DMF solution to remove the Fmoc-group. According to the sequence from the C-terminal end, 0.5M amino acid solution (solvent: DMF), 1.0M DIPEA (solvent: DMF&NMP) and 0.5M HBTU (solvent: DMF) were added to each well in amounts of 5, 10 and 5 equivalents, respectively, and reacted for 1-2 hours under a nitrogen atmosphere. After completion of each deprotection and coupling, each well was washed twice with DMF and twice with NMP. Even after coupling of the final amino acids, deprotection was performed to remove the Fmoc-group. The peptide synthesis was confirmed using the ninhydrin test method.

### Example 2: Screening of peptide by reaction with rhodamine-heparin-quenching dye

After dissolving 10 equivalents of rhodamine in DMSO, 1 equivalent of heparin sodium salt (Sigma) was repeatedly added to the solution three times, followed by reaction at room temperature for 4 hours. 1 equivalent of the heparin sodium salt having rhodamine bound thereto was reacted with 10 equivalents of EDC and 5 equivalents of NHS, and then 10 equivalents of a quenching dye (Black Hole Quencher-1, BHQ-1, Biosearch Technologies) was added thereto, followed by reaction at room temperature for 4 hours. The reaction mixture was dialyzed to remove unreacted rhodamine, EDC/NHS and quenching dye, followed by freeze drying.

The heparin having the rhodamine and quenching dye bound thereto was added to each well where the peptide was conjugated and reacted. After a predetermined time, each well was washed and observed under a fluorescence microscope to determine whether fluorescence was expressed therein. The bead in the well that showed the expression of fluorescence was separated and dissolved in THF or DCM, and then TFA cleavage cocktail was added thereto in an amount 20 ml per g of the resin. The mixture was shaken for 3 hours, and then filtered to separate the cocktail containing the resin and peptide dissolved therein. The filtered solution was evaporated using a rotary evaporator, and then cold ether was added thereto, or an excessive amount of cold ether was added directly to the TFA cocktail solution to crystallize the peptide into a solid. The solid was separated by centrifugation. The solid was washed several times with ether and centrifuged to completely remove the TFA cocktail. The obtained peptide was dissolved in distilled water, freeze-dried, and purified by liquid chromatography. The molecular weight of the purified peptide was analyzed by MALDI.

### Example 3: Examination of binding of synthesized peptide to heparin

In order to demonstrate that the synthesized peptide that showed the expression of fluorescence by binding to rhodamine-heparin-quenching dye in Example 2 actually binds to heparin, the following test was performed. The synthesized peptide was dissolved in PBS at various concentrations and coated on the surface of 96-well Maxisorp microtiter plates (Nunc). Then, it was blocked with 1% BSA at 37 °C for 1 hour, and sodium heparin (10 µg/100 µL) was added to the peptide and reacted with the peptide at room temperature for 4 hours. The plate was washed three times with PBS, and then horseradish peroxidase (HRP)-conjugated heparin binding protein (Lifespan Technologies, Salt Lake City, UT) diluted in PBS at a concentration of 1 µg/mL was reacted with the peptide at room temperature for 1 hour. To measure the binding of HRP-conjugated heparin binding protein to the peptide, ABTS was added to each well to develop color, and after 5 minutes, 1% SDS was added to each well to stop the reaction. Then, the absorbance of each well at 405 nm was measured. The absorbance of the peptide having the HRP-conjugated heparin binding protein bound thereto was calculated relative to 100% for the well surface coated with 1% BSA.

Binding % = (absorbance of well coated with heparin binding peptide/absorbance of well coated with BSA) X 100

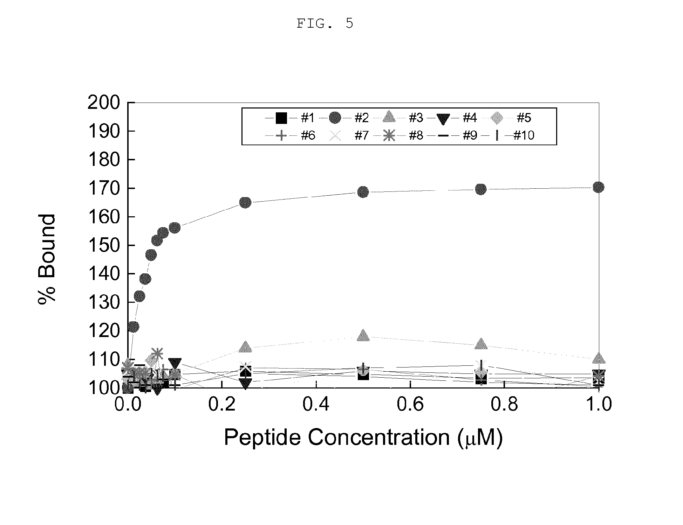

As a result, as shown in FIG. 5, the peptide of SEQ ID NO: 2 showed a high ability to bind to heparin, and the remaining peptides showed a low ability to bind to heparin.

### Example 4: Measurement of in vitro cell differentiation potential of heparin binding peptide

To measure the cell differentiation ability of the heparin binding peptide by calcium production, 1 x 10³ hMSCs (human mesenchymal stem cells) were dispensed into each well of a well-plate, and then treated with 10 mM of each of the peptides of SEQ ID NOS: 2 and 3 synthesized in Example 2. Then, the cells were cultured in a hard tissue forming medium (MSCBM medium containing 15% FBS (fetal bovine serum), 50 mg/mℓ L-ascorbic acid, 10⁻⁷ M dexamethasone, 1% antibiotic-antimycotic solution, and 10 mM beta-glycerol phosphate) for 14 days. After completion of the culture, the medium was removed, and the cells were washed twice with phosphate buffered saline (PBS). Then, the cells were fixed with 90% ethanol at 4 °C for 15 minutes and washed twice with distilled water, followed by staining with 2% Alizarin red S solution (pH 4.2; Alizarin red S powder, Junsei, JAPAN) for 5 minutes.

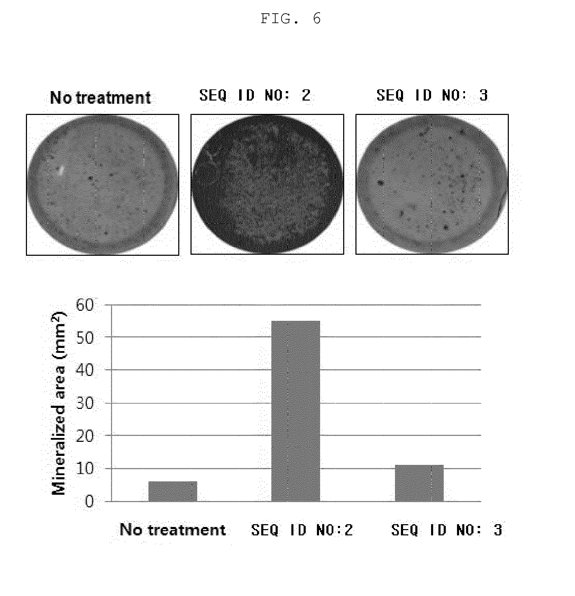

As a result, as shown in FIG. 6, the production of calcium was the highest in the cells treated with the heparin-binding peptide of SEQ ID NO: 2 and was the lowest in the cells treated with the peptide of SEQ ID NO: 3. This demonstrates that the peptide that binds to heparin has the ability to induce differentiation into bone tissue.

### INDUSTRIAL APPLICABILITY

As described above, the present invention makes it possible to discover a peptide consisting of 20 or more amino acids in a rapid manner, compared to conventional peptide discovering techniques. In addition, a bioactive peptide, which has a tissue regenerating effect and is capable of adhering to a specific biomaterial, can be discovered by PEPscovery (**PEP**tide Di**scovery**). The discovered bioactive peptide can be applied for the development of medical supplies, medical equipments and diagnostic chips.

Although the present invention has been described in detail with reference to the specific features, it will be apparent to those skilled in the art that this description is only for a preferred embodiment and does not limit the scope of the present invention. Thus, the substantial scope of the present invention will be defined by the appended claims and equivalents thereof.

## Claims

1. A method for discovering a bioactive peptide, the method comprising the steps of:
(a) adding bead resin to each well of a well plate, and synthesizing a peptide, which contains a protein fragment having desired bioactivity, on the surface of the bead in each well, thereby constructing a bioactive peptide library; and
(b) screening a peptide having desired bioactivity from the bioactive peptide library.

2. The method of claim 1, wherein the screening in step (b) is performed using an antibody or, a quenching dye and a fluorescent substance.

3. The method of claim 2, wherein the antibody in step (b) is a phosphatase- or peroxidase-conjugated antibody.

4. The method of claim 2, wherein the fluorescent substance in step (b) is selected from the group consisting of fluorescent substances having similar wavelengths to those of rhodamine and fluorescein isothiocyanate (FITC).
